# EUROPEAN PATENT SPECIFICATION

(11) **EP 0 714 647 B1**
(45) Date of publication and mention of the grant of the patent: **03.05.2000**
(21) Application number: 95119311.9
(22) Date of filing: 20.09.1991
(51) Int. Cl.: A61F 13/15

(54) **Sanitary absorbent article**
Hygienischer, saugfähiger Gegenstand
Garniture sanitaire absorbant

(30) Priority: 21.09.1990 US 586474
(43) Date of publication of application: 05.06.1996
(62) Divisional of application: 91116069.5
(73) Proprietor: KIMBERLY-CLARK WORLDWIDE, INC., Neenah, Wisconsin 54956 (US)
(72) Inventor: Gompel, Paul T. Van, Hortonville, Wisconsin 54944 (US); Suprise, Jody D., Neenah, Wisconsin 54956 (US); Schleinz, Robert J., Appleton, Wisconsin 54915 (US)
(74) Representative: Davies, Christopher Robert

(56) References cited:
- EP-A- 0 601 610
- WO-A-90/04374

## Description

This invention pertains to child's training pants.

Current absorbent articles, such as diapers, training pants, adult incontinence garments, and others, generally utilize a thermoplastic film as the liquid-impermeable backsheet of the article. Various elastic systems in the leg and waist areas are used to gather the article for fit and containment. These elastic systems and an absorbent structure are generally disposed between a liquid-impermeable thermoplastic backsheet and a liquid-permeable topsheet. This type of backsheet is thus liquid impervious, but plastic in feel and appearance.

Several backsheet designs have been developed to eliminate, as much as possible, the feel and appearance of plastic. One such backsheet design is a film-coated nonwoven. This backsheet comprises a thermoplastic film that is extruded onto a nonwoven web. The heat of the film and the pressure applied during the process provides the adhesion between the film and nonwoven layer. While the film does help to maintain the integrity of the nonwoven layer, it also results in a stiffer and less desirable backsheet.

Another backsheet design laminates the thermoplastic film to the nonwoven layer. The laminating is provided by applying an adhesive to the film or the nonwoven layer, and then attaching it to the other of the layers. Thus, in both this second design and the first one mentioned above, the thermoplastic film and the nonwoven layer are adhered totally along their mutually facing surfaces.

In order to overcome such drawbacks the invention provides an absorbent article according to independent claim 1. Further advantageous features and details of the absorbent article are evident from the dependent claims, the description and the drawings.

approach of defining the invention in general terms. The invention provides more specifically an absorbent article incorporating a poly baffle between the backsheet and absorbent structure to maintain the clothlike texture of the backsheet, while providing liquid impermeability.

The present invention generally comprises an absorbent article comprising a topsheet, an absorbent structure, and a backsheet. A baffle layer of material is disposed between the absorbent structure and the backsheet and is made of a material that is liquid impermeable, which also may be vapor permeable. The baffle layer is intermittently adhered to the backsheet at predetermined areas, thereby resulting in an absorbent article having a more clothlike texture.

The above-mentioned and other features and objects of this invention, and the manner of attaining them, will become more apparent and the invention itself will be better understood by reference to the following description of an embodiment of the invention taken in conjunction with the accompanying drawings, wherein:
Fig. 1 is a perspective view of an absorbent article incorporating the principles of the present invention;
Fig. 2 is a perspective, fragmentary view of a portion of the absorbent article incorporating the principles of the present invention; and
Fig. 3 is a cross-sectional view of a portion of the absorbent article incorporating the principles of the present invention.

The present invention provides several unique features to an absorbent article. One of these key features is a backsheet that has an improved clothlike texture. A second feature is the provision of a liquid-impermeable baffle between the absorbent structure and the backsheet, and which is intermittently or partially attached to the backsheet, thereby reducing the stiffness or rigidity that would be caused by totally adhering the backsheet and baffle together. A third feature of the present invention is the provision of the poly baffle not having to be continuous across the surface area of the backsheet or the absorbent structure.

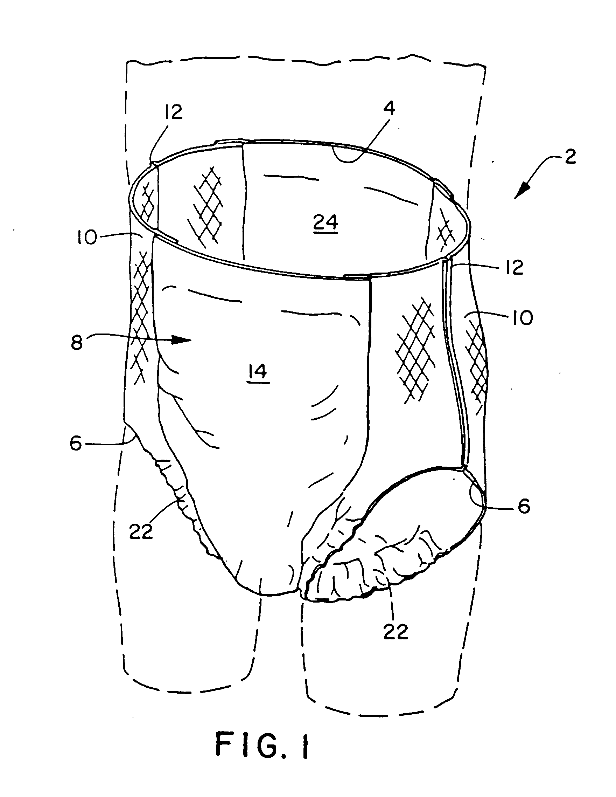

Referring to Fig. 1, there is illustrated a child's training pant 2 generally comprising a waist opening 4, leg openings 6, a central absorbent assembly 8, and stretchable side panels 10 connected to absorbent assembly 8 in order to define waist opening 4 and a leg opening 6. If desired, stretchable side panels 10 may be deleted and substituted by continuing or extending all or only portions of absorbent assembly 8, which is then joined together along seams 12 to define waist opening 4 and leg openings 6.

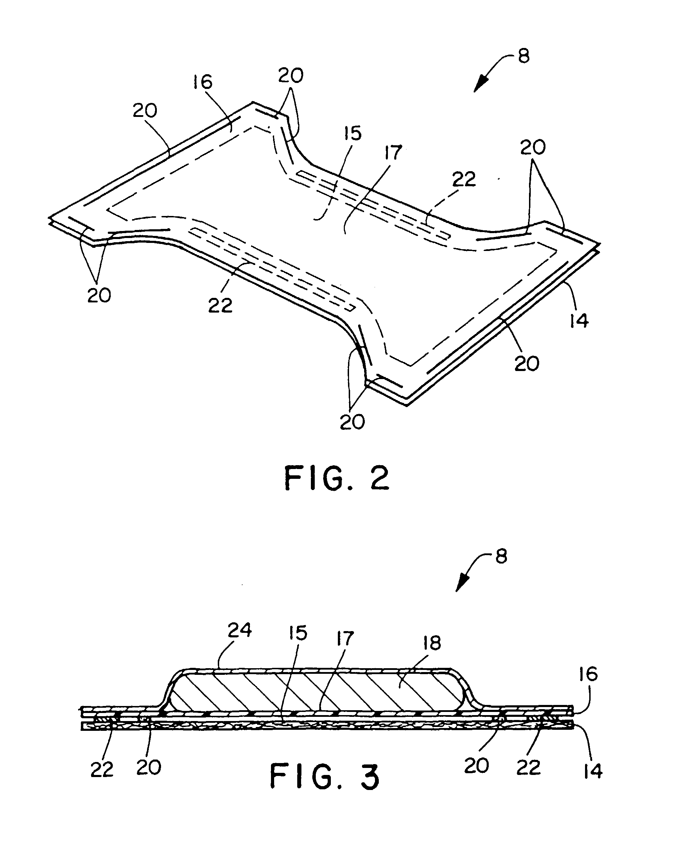

Referring to Fig. 2, a portion of absorbent assembly 8 is illustrated in a generally hourglass shape, which may be the shape of the absorbent assembly for training pant 2 or, for example, may be a portion of the absorbent assembly for a baby diaper. Backsheet 14, which can be made of any suitable liquid-permeable nonwoven web, has superposed thereon liquid-impermeable baffle layer 16. Shown in dotted lines is one shape for absorbent structure 18, which would be superposed on top of baffle layer 16. Thus, baffle layer 16 is sandwiched between backsheet 14 and absorbent structure 18.

Backsheet 14 and liquid-impermeable baffle layer 16 are intermittently or partially joined together at predetermined areas, depending upon the size and the type of the absorbent article, such that the common central portions 15, 17 of backsheet 14 and baffle layer 16 are unadhered together, i.e., freely moveable relative to one another. As one example, Fig. 2 illustrates a plurality of bond lines 20 that intermittently join together the adjacent peripheral areas of backsheet 14 and baffle layer 16. Thus, the mutually facing surfaces of backsheet 14 and baffle layer 16 that lie inwardly of bond lines 20 are free to move relative to each other, such as central portions 15, 17. It is this freedom of movement between nonwoven backsheet 14 and baffle layer 16 that provides the increased feel of clothlike texture to the absorbent article.

Fig. 2 also illustrates leg elastics 22 joined on either side of and between backsheet 14 and baffle layer 16. By joining leg elastics 22 in a stretched condition between backsheet 14 and baffle layer 16, there is not only provided the intermittent joining of backsheet 14 to baffle layer 16, but also a shirring or elastic gathering to the article.

Referring now to Fig. 3, there is illustrated a cross-sectional view of one absorbent article incorporating the present invention. Backsheet 14 and baffle layer 16 are joined along their peripheral edges by leg elastics 22, the joining of which serves to join backsheet 14 to baffle layer 16, and also to provide elasticity along the' edges. If desired, additional bond lines 20 can be provided either inboard or outboard of leg elastics 22, and in Fig. 3 bond lines 20 are illustrated inboard of leg elastics 22. Alternatively, when additional bond lines 20 are provided, leg elastics 22 can be joined to the side of baffle layer 16 that is opposite of backsheet 14, which generally would place elastics 22 between baffle layer 16 and topsheet 24. Disposed on top of baffle layer 16 is absorbent structure 18, which is also overlaid with liquid-permeable topsheet 24. Topsheet 24 is joined to baffle layer 16 and backsheet 14 in any suitable manner. As can be seen in Fig. 3, there is no attachment between backsheet 14 and baffle layer 16 between bond lines 20, thereby permitting backsheet 14 to have freedom of movement during placement of the absorbent article on a wearer, and during body movements of the wearer. This freedom of movement provides additional clothlike feel or texture to the article, which is highly desirable to both the wearer and, in the case of diapers and training pants, to mothers.

Although Fig. 2 illustrates baffle layer 16 congruent to backsheet 14, and also illustrates absorbent structure 18 lying within the peripheral confines of both backsheet 14 and baffle layer 16, various design configurations are contemplated by the invention. For example, baffle layer 16 may be varied in size such that it is actually smaller than the absorbent structure 18, so that selected portions of absorbent structure 18 lie directly adjacent breathable backsheet 14. Baffle layer 16 can also be multiple strips or layers of baffle material, rather than the single layer as illustrated. For example, baffle layer 16 can comprise a relatively small rectangular layer disposed in only the central portion 17 between absorbent structure 18 and backsheet 14, and a plurality of strips or segments of baffle material selectively positioned at preferred locations between absorbent structure 18 and backsheet 14.

Various configurations for attaching baffle layer 16 to backsheet 14 are also contemplated by the present invention. For example, bond lines 20 may be one continuous bond line along the peripheral portions between backsheet 14 and baffle layer 16. Alternatively, baffle layer 16 and backsheet 14 may be joined together by a plurality of bond lines that are substantially parallel running in either the machine direction or the cross direction, or can be joined together by a plurality of intersecting bond lines that form a grid-like design. The type of intermittent attachment between baffle layer 16 and backsheet 14 will generally be determined by the type of garment, size of garment, and the materials of which baffle layer 16 and backsheet 14 are made.

The bonded or joined surface area between backsheet 14 and baffle layer 16 is preferably between about 1% to about 30% of the mutually facing surface area between backsheet 14 and baffle layer 16, and more preferably between about 1% to about 15%.

Baffle layer 16 can be made of any suitable liquid-impermeable material, and preferably a polypropylene or polyethylene film having a thickness of about 25.4µm (about 1.0 mil), although thicknesses above 5.08 µm (0.20 mil) are suitable. Baffle layer 16 also can be made of materials that are suitably liquid impermeable or treated to be so. Examples are meltblown or film material made of polypropylene or polyolefin copolymers such as ethylene vinyl acetate, ethylene methyl acrylate, ethylene ethyl acrylate, polyvinyl chloride, and the like. Baffle layer 16 can also be made of a material that is not only liquid impermeable, but also vapor permeable. As a breathable, i.e., liquid-impermeable, vapor-permeable material, baffle layer 16 has a water vapor transmission rate value of at least about 2,000 g/m²/24 hours. Preferably, baffle layer 16 has a water vapor transmission rate value of at least about 4,000 g/m²/24 hours, and more preferably a value of at least about 5,000 g/m²/24 hours. Baffle layer 16 can also be selectively breathable, in that portions may be breathable, while other portions are impermeable both to liquid and vapor. It is preferred that the breathable area of baffle layer 16 be about 20 cm ² or greater.

A breathable baffle layer 16 can comprise a microporous polymer film, such as Grade TMP-1 film manufactured by Mitzui Toatsu Chemical, Inc., Tokyo, Japan; or a nonwoven fibrous material, such as a spunbonded or meltblown web composed of synthetic polymer fibers that are treated to be liquid impermeable and vapor permeable.

Both topsheet 24 and breathable backsheet 14 can be made of similar or different materials that are liquid permeable, such as a spunbonded web composed of synthetic polymer filaments; a spunlace web; a bonded-carded web composed of synthetic polymer fibers, and the like. Suitable synthetic polymers include polyethylene, polypropylene, polyester, and nylon. In one embodiment, the polymer filaments have a tex (denier) within the range of about 0.167 (1.5) to about 0.778 (7) tex (d) The filaments are arranged to form a layer having a basis weight within the range from about 10 grams/m² to about 35 grams/m².

Absorbent structure 18 can comprise various types and mixtures of fibrous material. For example, absorbent structure 18 may comprise cellulosic fluff, synthetic fibers, absorbent gelling materials in the form of particles, fibers, layers and the like, and various mixtures or blends thereof.

## Claims

1. A disposable child's training pant, comprising:
an absorbent assembly (8) comprising a liquid permeable topsheet (24), a liquid permeable nonwoven backsheet (14), an absorbent (18) disposed between said topsheet (24) and said backsheet (14), seams (12) that form a waist opening (4) and a pair of leg openings (6), and
a wholly liquid impermeable baffle layer (16) positioned between said backsheet (14) and said absorbent (18), the absorbent lying within the peripheral confines of both the backsheet (14) and the baffle layer (16), said liquid impermeable baffle layer (16) and said backsheet (14) being joined together by a continuous bond line or intermittent bond lines along their adjacent peripheral areas so that their common central portions (15, 17) freely move relative to each other.

2. The pant of claim 1 wherein stretchable side panels (10) are joined to said absorbent assembly (8) and
said seams (12) join said side panels (10) to form said waist opening (4) and said pair of leg openings (6).

3. The pant of any of the preceding claims wherein said backsheet (14) and said baffle layer (16) are congruent.

4. The pant of any of the preceding claims wherein said baffle layer (16) is smaller in dimension than said backsheet (14).

5. The pant of any of the preceding claims wherein said baffle layer (16) is smaller in dimension than said absorbent (18).

6. The pant of any of the preceding claims wherein said baffle layer (16) is liquid impermeable and vapor permeable, and has a water vapor transmission rate value of at least about 2,000 grams/square meter/24 hours.

7. The pant of claim 6 wherein said baffle layer (16) is vapor permeable in selected portions thereof.

8. The pant of claim 7 wherein said selected vapor permeable portions have a total area of about 20 square centimeters or greater.

9. The pant of any of the preceding claims wherein said bond line is a line of elastic material.

## Patentansprüche

1. Kinderwegwerfwindelhose, umfassend:
einen saugfähigen Artikel (8), seinerseits umfassend eine flüssigkeitsdurchlässige Deckfolie (24), eine flüssigkeitsdurchlässige Vlies-Bodenfolie (14), einen Saugkörper (18), der zwischen der Deckfolie (24) und der Bodenfolie (14) angeordnet ist, Säume (12), die eine Taillenöffnung (4) und ein Paar Beinöffnungen (6) ausbilden und
eine völlig flüssigkeitsundurchlässige Ablenkschicht (16), die zwischen der Bodenfolie (14) und dem Saugkörper (18) angeordnet ist, wobei der Saugkörper innerhalb der Umfangskonturen sowohl der Bodenfolie (14) als auch der Ablenkschicht (16) liegt, wobei die flüssigkeitsundurchlässige Ablenkschicht (16) und die Bodenfolie (14) miteinander durch eine ununterbrochene Klebespur oder unterbrochene Klebespuren entlang ihrer benachbarten Umfangsbereiche verbunden sind, so daß ihre gemeinsamen mittigen Abschnitte (15, 17) sich relativ zueinander frei bewegen.

2. Windelhose nach Anspruch 1, wobei dehnbare Seitenstreifen (10) zu dem saugfähigen Aufbau (8) zusammengefügt sind und
die Säume (12) die Seitenstreifen (10) verbinden, um die Taillenöffnung (4) und das Paar Beinöffnungen (6) zu bilden.

3. Windelhose nach jedem der vorangehenden Ansprüche, wobei die Bodenfolie (14) und die Ablenkschicht (16) deckungsgleich sind.

4. Windelhose nach jedem der vorangehenden Ansprüche, wobei die Ablenkschicht (16) kleiner in den Abmessungen als die Bodenfolie (14) ist.

5. Windelhose nach jedem der vorangehenden Ansprüche, wobei die Ablenkschicht (16) kleiner in den Abmessungen als der Saugkörper (18) ist.

6. Windelhose nach jedem der vorangehenden Ansprüche, wobei die Ablenkschicht (16) flüssigkeitsundurchlässig und dampfdurchlässig ist und einer Wasserdampfdurchsatzratenwert von zumindest ungefähr 2.000 Gramm/Quadratmeter/24 Stunden aufweist.

7. Windelhose nach Anspruch 6, wobei die Ablenkschicht (16) in ausgewählten Abschnitten davon dampfdurchlässig ist.

8. Windelhose nach Anspruch 7, wobei die ausgewählten dampfdurchlässigen Abschnitte eine Gesamtfläche von ungefähr 20 Quadratzentimetern oder mehr aufweisen.

9. Windelhose nach jedem der vorangehenden Ansprüche, wobei die Klebespur ein Streifen aus elastischem Material ist.

## Revendications

1. Culotte d'apprentissage de la propreté jetable pour enfant, comprenant :
un ensemble absorbant (8) comprenant une feuille supérieure perméable aux liquides (24), une feuille de fond non tissée perméable aux liquides (14), un matériau absorbant (18) disposé entre ladite feuille supérieure (24) et ladite feuille de fond (14), des coutures (12) qui forment une ouverture de ceinture (4) et une paire d'ouvertures de jambe (6), et
une couche formant barrière (16) complètement imperméable aux liquides positionnée entre ladite feuille de fond (14) et ledit matériau absorbant (18), le matériau absorbant s'étendant à l'intérieur des limites périphériques à la fois de la feuille de fond (14) et de la couche formant barrière (16), ladite couche formant barrière (16) imperméable aux liquides et ladite feuille de fond (14) étant réunies ensemble par une ligne de liaison continue ou des lignes de liaison intermittentes, le long de leurs zones périphériques adjacentes, de telle sorte que leurs portions centrales communes (15,17) se déplacent librement l'une par rapport à l'autre.

2. Culotte selon la revendication 1, dans laquelle des panneaux latéraux extensibles (10) sont réunis audit ensemble absorbant (8) et
lesdites coutures (12) réunissent lesdits panneaux latéraux (10) pour former ladite ouverture de ceinture (4) et ladite paire d'ouvertures de jambe (6).

3. Culotte selon l'une quelconque des revendications précédentes, dans laquelle ladite feuille de fond (14) et ladite couche formant barrière (16) sont de mêmes forme et taille.

4. Culotte selon l'une quelconque des revendications précédentes, dans laquelle ladite couche formant barrière (16) a des dimensions plus petites que ladite feuille de fond (14).

5. Culotte selon l'une quelconque des revendications précédentes, dans laquelle ladite couche formant barrière (16) a des dimensions plus petites que ledit matériau absorbant (18).

6. Culotte selon l'une quelconque des revendications précédentes, dans laquelle ladite couche formant barrière (16) est imperméable aux liquides et perméable à la vapeur, et a une valeur de débit de transmission de vapeur d'eau d'au moins environ 2.000 g/m²/24 h.

7. Culotte selon la revendication 6, dans laquelle ladite couche formant barrière (16) est perméable à la vapeur dans des portions sélectionnées de celle-ci.

8. Culotte selon la revendication 7, dans laquelle lesdites portions sélectionnées perméables à la vapeur ont une surface totale d'environ 20 cm² ou plus.

9. Culotte selon l'une quelconque des revendications précédentes, dans laquelle ladite ligne de liaison est une ligne de matériau élastique.
